# EUROPÄISCHE PATENTSCHRIFT

(11) **EP 0 830 868 B1**
(45) Veröffentlichungstag und Bekanntmachung des Hinweises auf die Patenterteilung: **25.09.2002**
(21) Anmeldenummer: 97111016.8
(22) Anmeldetag: 02.07.1997
(51) Int. Cl.: A61M 5/28, A61M 5/34

(54) **Vorgefüllte Spritze für medizinische Zwecke**
Prefilled syringe for medical purpose
Seringue préremplie pour but médical

(30) Priorität: 23.09.1996 DE 19638940
(43) Veröffentlichungstag der Anmeldung: 25.03.1998
(73) Patentinhaber: Arzneimittel GmbH Apotheker Vetter & Co. Ravensburg, D-88212 Ravensburg (DE)
(72) Erfinder: Vetter, Helmut, 88212 Ravensburg (DE); Vetter, Udo J., 88214 Ravensburg (DE); Otto, Thomas, 88250 Weingarten (DE); Rössling, Georg, Dr., 13465 Berlin (DE)
(74) Vertreter: Dziewior, Joachim, Dipl.-Phys. Dr.

(56) Entgegenhaltungen:
- EP-A- 0 412 283
- EP-A- 0 440 846
- EP-A- 0 685 237
- FR-A- 2 259 624
- GB-A- 1 108 900
- US-A- 2 677 374
- US-A- 3 424 155
- US-A- 4 568 336
- US-A- 5 354 286

## Beschreibung

Die Erfindung ist in Anspruch 1 definiert und betrifft eine vorgefüllte Spritze für medizinische Zwecke mit einem Spritzenzylinder und einem darin angeordneten, mittels einer Kolbenstange verschiebbaren Spritzenkolben, ferner mit einem am kanülenseitigen Ende des Spritzenzylinders axial aufgesetzten, im Rastsitz gehaltenen Kanülenansatzstück, das das kanülenseitige Ende des Spritzenzylinders umgreift, sowie mit einem zum Kanülenansatzstück koaxialen, in den Spritzenzylinder eingesetzten Verschlußstopfen, der einen einerseits stirnseitig dem Spritzenzylinder und andererseits einer Ringschulter des Kanülenansatzstücks anliegenden Ringbund aufweist und mit einer axial verlaufenden Durchgangsbohrung versehen ist.

Eine derartige Spritze ist beispielsweise aus der DE 41 27 650 bekannt. Bei dieser Spritze ist auf das Kanülenansatzstück ein Tip Cap aufgesetzt, das die Durchgangsbohrung des Verschlußstopfens stirnseitig abdichtet, wozu der Verschlußstopfen ein Ansatzstück aufweist, das sich in den Konus des Kanülenansatzstücks hinein erstreckt. Die Abdichtung des in der vorgefüllten Spritze befindlichen Medikaments erfolgt somit über die Dichtfläche zwischen dem Tip Cap und dem Ansatzstück, was in Einzelfällen nicht ausreichend sein kann.

US-A-4 747 839 zeigt die Merkmale im Oberbegriff vom Anspruch 1.

Der Erfindung liegt auch die Aufgabe zugrunde, eine vorgefüllte Spritze der eingangs gemannten Art so auszubilden, daß das Medikament während der Lagerung steril eingeschlossen ist und nur mit dem Material des Spritzenzylinders, Verschlußstopfens und des Spritzenkollbens, vorzugswweise also nur mit Gummi und Glas in Berührung kommt.

Diese Aufgabe wird nach der Erfindung dadurch gelöst, daß in der Durchgangsbohrung eine diese verschließende elastische Membran angeordet ist, der auf der zum Spritzenzylinder abgewandten Seite wenigstens ein mit seiner Spitze zur Membran hin gerichteter Dorn mit geringem Abstand gegenübersteht.

Der durch die Erfindung erreichte Vorteil besteht im wesentlichen darin, daß der Verschlußstopfen durch die, in der Durchgangsbohrung angeordnete Membran vollständig abgedichtet ist, so daß das Medikament nicht mit dem in die Durchgangsbohrung des Verschlußstopfens üblicherweise hineinragenden Stift des Tip Caps in Berührung kommt. Um die Membran vor der Injektion zu öffnen, genügt es, den Spritzenkolben mittels der Kolbenstange geringfügig zum kanülenseitigen Ende hin zu verstellen, wodurch der Druck im Inneren des Spritzenzylinders so hoch wird, daß die Membran zur Spitze des Dorns hin bewegt wird, wodurch die Membran angestochen wird.

Um dieses Öffnen der Membran reproduzierbar zu gestalten, sieht die Erfindung in bevorzugter Ausgestaltung vor, daß die Kolbenstange in ihrem an den Kolben angrenzenden Bereich mit einem kurzen, über den Kolbenschaft radial vorstehenden Außengewinde versehen ist, zu dem ein Innengewinde an dem dem Kanülenansatzstück abgewandten Ende des Spritzenzylinders bzw. an der Fingerauflage korrespondiert. Die Anordnung sowie die Länge des Außengewindes ist dabei so gewählt, daß der sich im Inneren des Spritzenzylinders aufbauende Druck gerade ausreicht, um die Membran bis zur Spitze des Dorns hin zu bewegen. Anschließend kann der Gewindeteil der Kolbenstange aus dem Innengewinde frei kommen, so daß die Kolbenstange vom Anwender frei bewegt werden kann.

In weiter bevorzugter Ausführungsform der Erfindung weist der Verschlußstopfen auf seiner dem Spritzenzylinder abgewandten Seite eine sacklochförmige Ausnehmung auf, in der ein den Dorn tragendes Ringelement formschlüssig eingesetzt ist. Dieses Ringelement kann insbesondere aus vergleichsweise formsteifem Kunststoff bestehen. Die Außenmantelfläche des Ringelementes weist zweckmäßigerweise die Gestalt eines Doppelkonus auf, wodurch ein selbsttätiger Halt in der Ausnehmung gewährleistet ist. Die Konusflächen können sich dabei zur einfacheren Montage vorzugsweise zu den Stirnflächen des Ringelements hin verjüngen.

Weiter ist es zweckmäßig, wenn die dem Dorn zugekehrte Fläche der Membran bündig zum Boden der Ausnehmung vorläuft. Auf diese Art und Weise wird der kürzestmögliche Abstand der Membran zum Dorn hin erreicht.

In weiter vorteilhafter Ausgestaltung der Erfindung ist auf der der Membran abgekehrten Seite des Dorns eine Filterscheibe in der Durchgangsbohrung angeordnet. Diese Filterscheibe dient nicht nur dazu, evtl. im flüssigen Medikament vorhandene Feststoffpartikel zurückzuhalten, sondern gegebenenfalls auch Teile der Membran, die sich bei deren Öffnen lösen können. Durch die Anordnung der Filterscheibe ist zunächst gewährleistet, daß diese während der Lagerung und bei durch den Transport auftretenden Druckunterschieden nicht beschädigt werden kann. Darüberhinaus kommt die Filterscheibe während der Lagerung nicht in Kontakt mit dem Medikament, wird also während dieser gesamten Zeit trocken gehalten. Die Filterscheibe bleibt darüber hinaus steril, da sie auf der einen Seite von der Membran und auf der anderen Seite durch das Tip Cap geschützt ist. Die Anordnung des Filters im Inneren des Ringelements hat darüber hinaus den Vorteil, daß sie vom Patienten nicht zu erkennen ist. Dadurch wird bei ihm, im Gegensatz zur herkömmlichen Anwendung, bei der ein externer Filter montiert werden muß, kein Mißtrauen gegenüber dem Medikament erweckt. Im übrigen wird hierdurch ausgeschlossen, daß, sofern das Vorhandensein einer Filterscheibe in jedem Fall erforderlich ist, die Anbringung vor der Injektion nicht unbeabsichtigt vergessen werden kann.

Weiter wird im Rahmen der Erfindung vorgeschlagen, daß das Kanülenansatzstück mit einem hülsenförmigen Anschlußteil das mit einem Außenbund versehene kanülenseitige Ende des Spritzenzylinders umgreift.

Bei Ausbildung der Spritze beispielsweise als Doppelkammerspritze, bei der in der kanülenseitigen Kammer das Präparat in lyophilisierter Form sich befindet und die Lyophilisierung in der Spritze erfolgt, kann im Rahmen der Erfindung vorgesehen sein, daß am Anschlußteil ein radial einwärts vorstehender Ringvorsprung angeordet ist, und daß am Außenbund des Spritzenzylinders zwei in axialer Richtung hintereinander angeordnete Ringnuten vorgesehen sind, wobei das Kanülenansatzstück bei in die erste Ringnut greifendem Ringvorsprung den Verschlußstopfen in einer, einen Ringspalt zwischen der Innenseite des Außenbundes und dem Verschlußstopfen bildenden Lage hält, während der Verschlußstopfen bei in die zweite ringnutgreifendem Ringvorsprung der Innenseite des Außenbundes dichtend anliegt. Hierdurch besteht die Möglichkeit, über den Ringspalt zunächst die Lyophilisierung vorzunehmen und im Anschluß daran, das Kanülenansatzstück vollständig auf den Spritzenzylinder aufzudrücken, wodurch der Ringvorsprung von der ersten in die zweite Ringnut übergeht.

Zum einfacheren Aufsetzen des Anschlußteils kann dieses vorteilhafterweise mit mehreren, über den Umfang gleichmäßig verteilt angeordeten Ausnehmungen versehen sein, die als axial verlaufende, sich bis zum freien Rand des Anschlußteiles ersteckende Schlitze ausgebildet sind. Um hierbei ein selbsttätiges Lösen zu vermeiden, empfiehlt es sich, daß das Kanülenansatzstück durch einen das Anschlußteil umgreifenden Sicherungsring fixiert ist.

Im übrigen kann das Kanülenansatzstück zweckmäßigerweise einen durch ein Tip Cap geschlossenen Konus zum Aufsetzen der Kanüle aufweisen.

Schließlich ist es zur Überprüfung, ob sich die Spritzen noch in ihrem Originalzustand befindet, von Vorteil, wenn am Sicherungsring über einen als Sollbruchstelle ausgebildeten Anschlußsteg eine bei auf das Anschlußteil aufgeschobenen Sicherungsring den Konus für die Kanüle bzw. das darauf aufgesetzte Tip Cap umschließende Sicherungskappe angeschlossen ist.

Im folgenden wird die Erfindung an einem in der Zeichnung dargestellten Ausführungsbeispiel näher erläutert; es zeigen:

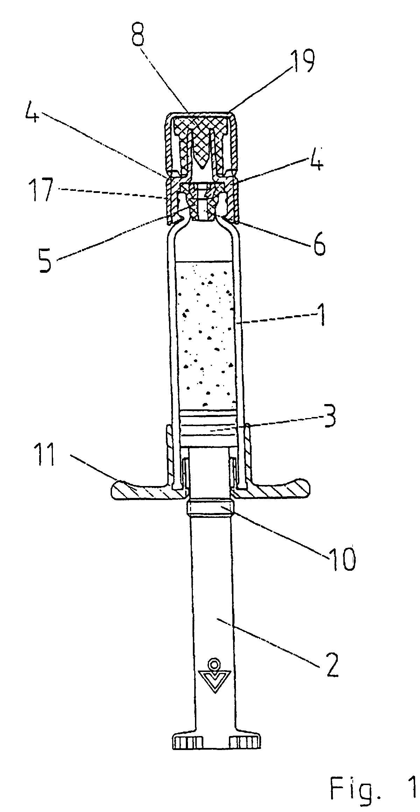

- Fig. 1: eine Spritze nach der Erfindung in Gesamtansicht,

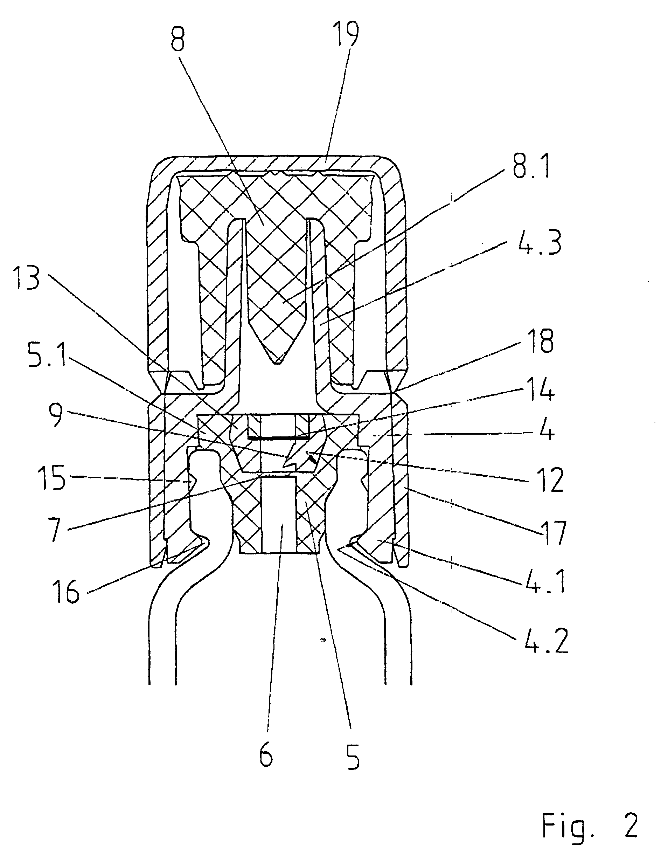

- Fig. 2: das kanülenseitige Ende der Spritze nach Fig. 1 in vergrößerter Darstellung,
- Fig. 3: den Verschlußverstopfen der Spritze in nochmals vergrößerter Darstellung.

Die in der Zeichnung dargestellte Spritze ist dazu vorgesehen, in vorgefülltem Zustand für die Anwendung bereitgestellt zu werden. Die Spritze besteht im einzelnen aus einem Spritzenzylinder 1 und einem darin angeordneten, mittels einer Kolbenstange 2 verschiebbaren Spritzenkolben 3. Am kanülenseitigen Ende des Spritzenzylinders 1 ist axial ein im Rastsitz gehaltenes Kanülenansatzstück 4 aufgesetzt, das das kanülenseitige Ende des Spritzenzylinders 1 umgreift. Weiter ist in den Spritzenzylinder 1 ein Verschlußstopfen 5 eingesetzt, der koaxial zum Kanülenansatzstück 4 angeordnet ist und einen Ringbund 5.1 aufweist, der einerseits stirnseitig dem Spritzenzylinder 1 und andererseits einer Ringschulter des Kanülenansatzstücks 4 anliegt. Dieser Verschlußstopfen 5 ist mit einer axial verlaufenden Durchgangsbohrung 6 versehen.

In der Durchgangsbohrung 6 ist eine diese verschließende elastische Membran 7 angeordet, durch die sichergestellt ist, daß das Medikament nicht beispielsweise mit dem dahinter liegenden Stift 8.1 des Tip 8 Caps in Berührung kommt. Vielmehr bildet der Verschlußstopfen 5 eine vollständige Abdichtung zum Kanülenansatzstück hin.

Auf der dem Spritzenzylinder 1 abgewandten Seite steht der Membran 7 ein ihr mit seiner Spitze entgegengerichteter Dorn 9 mit geringem Abstand gegenüber, der dem Öffnen der Membran 7 vor der Anwendung der Spritze dient. Dazu wird vor der Anwendung der Spritzenkolben 3 zum kanülenseitigen Ende des Spritzenzylinders 1 hin verschoben, wodurch sich der Druck im Inneren des Spritzenzylinders 1 erhöht. Dadurch wird die Membran 7 zum Dorn 9 hin "ausgebeult", bis sie die Spitze des Dorns 9 berührt und dadurch angestochen wird. Hierdurch bildet sich in der Regel ein Riß über die gesamte Querschnittsfläche der Durchgangsbohrung 6. Anschließend kann die Spritze entlüftet und sodann die Injektion vorgenommen werden.

Um die Druckerhöhung im Inneren des Spritzenzylinders 1 kontrolliert vornehmen zu können, ist die Kolbenstange 2 an ihrem an den Kolben 3 angrenzenden Bereich mit einem kurzen, über den Kolbenschaft radial vorstehenden Außengewinde 10 versehen. Dieses Außengewinde 10 korrespondiert mit einem Innengewinde an der Fingerauflage 11, wobei die Anordnung des Außengewindes 10 und dessen Länge so gewählt ist, daß der sich beim Eindrehen der Kolbenstange 2 aufbauende Druck gerade ausreicht, um die Membran 7 zu zerstören. Im Anschluß daran kann die Kolbenstange 2 frei bewegt werden um - wie schon ausgeführt - die Entlüftung und sodann die Injektion durchführen zu können.

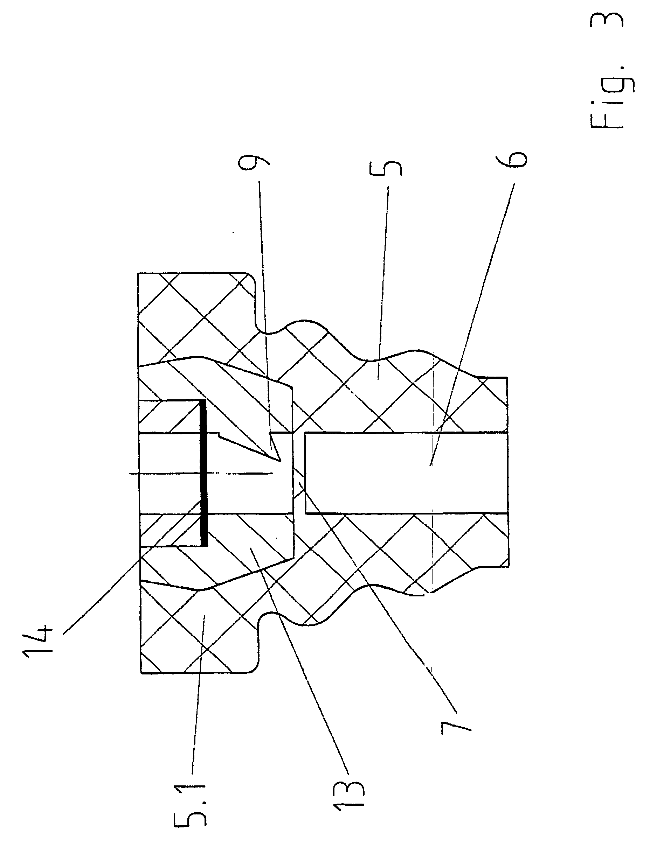

Der Verschlußstopfen 5 weist auf seiner dem Spritzenzylinder 1 abgewandten Seite eine sacklochförmige Ausnehmung 12 auf, in der ein den Dorn 9 tragendes Ringelement 13 formschlüssig eingesetzt ist. Dieses Ringelement 13 besteht aus gegenüber dem Verschlußstopfen 5 härter eingestelltem Material, also beispielsweise aus Kunststoff. Die Außenmantelfläche des Ringelements 13 weist, wie insbesondere die Fig. 3 gut erkennen läßt, die Gestallt eines Doppelkonus auf, wobei die Konusflächen sich zu den Stirnflächen des Ringelements 13 hin verjüngen. Die dem Dorn 9 zugekehrte Fläche der Membran 7 verläuft, wie sich ebenfalls aus Fig. 3 erkennen läßt, bündig zum Boden der Ausnehmung 12.

Auf der der Membran 7 abgekehrten Seite des Dorns 9 ist eine Filterscheibe 14 in der Durchgangsbohrung 6 angeordnet. Diese Filterscheibe 14 sorgt dafür, daß eventl. im Medikament vorhandene feste Partikel oder auch Teile der durchstoßenen Membran 7 nicht mit injiziert werden. Die Anordnung der Filterscheibe bietet dabei den Vorteil, daß sie zunächst während der Lagerung der Spritze trocken und darüberhinaus steril eingeschlossen bleibt. Die Anordnung innerhalb des Ringelements 13 bietet den Vorteil, daß die Filterscheibe 14 vom Patienten nicht zu erkennen ist, so daß auch kein - in der Regel ohnehin unbegründetes - Mißtrauen gegenüber dem Medikament erweckt wird. Durch die von vorn herein eingebrachte Filterscheibe 14 ist im übrigen sichergestellt, daß deren Anbringung vor der Injektion nicht versehentlich vergessen werden kann.

Das Kanülenansatzstück 4 ist im übrigen mit einem hülensförmigen Anschlußteil 4.1 versehen, das das mit einem Außenbund versehene kanülenseitige Ende des Spritzenzylinders 1 umgreift. Am Anschlußteil 4.1 ist dabei ein radial einwärtsvorstehender Ringvorsprung 4.2 angeordnet, der in einen von zwei am Außenbund des Spritzenzylinders 1 in axialer Richtung hintereinander angeordete Ringnuten 15,16 ein eingreift. Bei in die erste Ringnut 15 greifendem Ringvorsprung 4.2 hält das Kanülenansatzstück 4 den Verschlußstopfen 5 in einer einen Ringspalt zwischen der Innenseite des Außenbundes und dem Verschlußstopfen 5 bildenden Lage. Über diesen Ringspalt kann beispielsweise bei einer Doppelkammerspritze das in die kanülenseitige Kammer eingebrachte Medikament lyophilisiert und im Anschluß daran der Ringspalt dadurch geschlossen werden, daß das Kanülenansatzstück 4 vollständig auf den Spritzenzylinder 1 aufgeschoben wird, bis der Ringvorsprung 4.2 in die zweite Ringnut 16 eingreift. Dann liegt der Verschlußstopfen 5 der Innenseite des Außenbundes dichtend an.

Das Anschlußteil 4.1 ist in in der Zeichnung nicht näher dargesteller Weise zweckmäßigerweise mit mehreren Ausnehmungen versehen, die gleichmäßig über den Umfang verteilt angeordet sind. Diese Ausnehmungen sind als axial verlaufende, sich bis zum freihen Rand des Anschlußteiles 4.1 erstreckende Schlitze ausgebildet. Hierdurch wird die Montage des Kanülenansatzstücks 4 wesentlich erleichtert. Um hierbei ein selbsttätiges oder unbeabsichtigtes Lösen des Anschlußteils 4.1 zu verhindern, ist das Kanülenansatzstück 4 durch einen das Anschlußteil 4.1 umgreifenden Sicherungsring 17 fixiert. Am Sicherungsring 17 ist über einen als Sollbruchstelle ausgebildeten Anschlußsteg 18 eine Sicherungskappe 19 angeschlossen, die den Konus 4.3 für die Kanüle bzw. das darauf aufgesetzte Tip Cap 8 umschließt. Das Vorhandensein dieser Sicherungskappe 19 gewährleistet, daß sich die vorgefüllte Spritze vor Beginn der Injektion noch in ihrem Originalzustand befindet.

## Patentansprüche

1. Vorgefüllte Spritze für medizinische Zwecke, mit einem Spritzenzylinder (1) und einem darin angeordneten, mittels einer Kolbenstange (2) verschiebbaren Spritzenkolben (3), ferner mit einem am kanülenseitigen Ende des Spritzenzylinders (1) axial aufgesetzten, in einem Rastsitz (15, 16) gehaltenen Kanülenansatzstück (4), das das kanülenseitige Ende des Spritzenzylinders (1) umgreift, sowie mit einem zum Kanülenansatzstück (4) koaxialen, in den Spritzenzylinder (1) eingesetzten Verschlußstopfen (5), der einen einerseits stirnseitig dem Spritzenzylinder (1) und andererseits einer Ringschulter des Kanülenansatzstücks (4) anliegenden Ringbund (5.1) aufweist und mit einer axial verlaufenden Durchgangsbohrung (6) versehen ist, wobei in der Durchgangsbohrung (6) eine diese verschließende elastische Membran (7) angeordnet ist, der auf der dem Spritzenzylinder (1) abgewandten Seite wenigstens ein mit seiner Spitze zur Membran (7) hin gerichteter Dorn (9) mit geringem Abstand gegenübersteht, **dadurch gekennzeichnet, daß** der Verschlußstopfen (5) auf seiner dem Spritzenzylinder (1) abgewandten Seite eine sacklochförmige Ausnehmung (12) aufweist, in der ein den Dorn (9) tragendes Ringelement (13) formschlüssig eingesetzt ist.

2. Spritze nach Anspruch 1, **dadurch gekennzeichnet, daß** die Außenmantelfläche des Ringelements (13) die Gestalt eines Doppelkonus aufweist.

3. Spritze nach Anspruch 2, **dadurch gekennzeichnet, daß** die Konusflächen sich zu den Stirnflächen des Ringelements (13) hin verjüngen.

4. Spritze nach einem der Ansprüche 1 bis 3, **dadurch gekennzeichnet, daß** die dem Dorn (9) zugekehrte Fläche der Membran (7) bündig zum Boden der Ausnehmung (12) verläuft.

5. Spritze nach einem der Ansprüche 1 bis 4, **dadurch gekennzeichnet, daß** die Kolbenstange (2) in ihrem an den Kolben (3) angrenzenden Bereich mit einem kurzen, über den Kolbenschaft radial vorstehenden Außengewinde (10) versehen ist, zu dem ein Innengewinde am dem Kanülenansatzstück (4) abgewandten Ende des Spritzenzylinders (1) bzw. an einer Fingerauflage (11) korrespondiert.

6. Spritze nach einem der Ansprüche 1 bis 5, **dadurch gekennzeichnet, daß** auf der der Membran (7) abgekehrten Seite des Dorns (9) eine Filterscheibe (14) in der Durchgangsbohrung (6) angeordnet ist.

7. Spritze nach einem der Ansprüche 1 bis 6, **dadurch gekennzeichnet, daß** das Kanülenansatzstück (4) mit einem hülsenförmigen Anschlußteil (4.1) das mit einem Außenbund versehene kanülenseitige Ende des Spritzenzylinders (1) umgreift.

8. Spritze nach einem der Ansprüche 1 bis 7, **dadurch gekennzeichnet, daß** am Anschlußteil (4.1) ein radial einwärts vorstehender Ringvorsprung (4.2) angeordnet ist, und daß am Außenbund des Spritzenzylinders (1) zwei in axialer Richtung hintereinander angeordnete Ringnuten (15,16) vorgesehen sind, wobei das Kanülenansatzstück (4) bei in die erste Ringnut (15) greifendem Ringvorsprung (4.2) den Verschlußstopfen (5) in einer einen Ringspalt zwischen der Innenseite des Außenbundes und dem Verschlußstopfen (5) bildenden Lage hält, während der Verschlußstopfen bei in die zweite Ringnut (16) greifendem Ringvorsprung (4.2) der Innenseite des Außenbundes dichtend anliegt.

9. Spritze nach einem der Ansprüche 1 bis 8, **dadurch gekennzeichnet, daß** das Anschlußteil (4.1) mit mehreren, über den Umfang gleichmäßig verteilt angeordneten Ausnehmungen versehen ist, die als axial verlaufende, sich bis zum freien Rand des Anschlußteiles (4.1) erstreckende Schlitze ausgebildet sind.

10. Spritze nach einem der Ansprüche 1 bis 9, **dadurch gekennzeichnet, daß** das Kanülenansatzstück (4) durch einen das Anschlußteil (4.1) umgreifenden Sicherungsring (17) fixiert ist.

11. Spritze nach einem der Ansprüche 1 bis 10, **dadurch gekennzeichnet, daß** das Kanülenansatzstück (4) einen durch ein Tip-Cap (8) geschlossenen Konus (4.3) zum Aufsetzen der Kanüle aufweist.

12. Spritze nach Anspruch 11, **dadurch gekennzeichnet, daß** am Sicherungsring (17) über einen als Sollbruchstelle ausgebildeten Anschlußsteg (18) eine bei auf das Anschlußteil (4.1) aufgeschobenen Sicherungsring (17) den Konus für die Kanüle bzw. das darauf aufgesetzte Tip-Cap (8) umschließende Sicherungskappe (19) angeschlossen ist.

## Claims

1. Pre-filled syringe for medical purposes, with a syringe barrel (1) and a syringe plunger (3) which is arranged inside it and which can be pushed by means of a plunger rod (2); furthermore with a needle annex piece (4) which is positioned axially on the needle end of the syringe barrel (1) and is held in a snap-in receiver (15, 16), and which encompasses the needle end of the syringe barrel (1); as well as with a sealing plug (5) which is co-axial to needle annex piece (4) and is inserted in the syringe barrel (1), and which has a ring collar (5.1) which on the one hand rests frontally against the syringe barrel (1) and on the other hand rests against a ring shoulder of the needle annex piece (4), and is equipped with a drilled through-hole (6) which runs axially,
wherein located in the drilled through-hole (6) is an elastic membrane (7) which seals this off, and which - on the side facing away from the from the syringe barrel (1) - lies opposite at least one spike (9) whose tip is directed towards the membrane (7), a short distance from it,
**characterised in that** on its side facing away from the syringe barrel (1), the sealing plug (5) has a recess (12) in the shape of a blind hole, in which a ring element (13) which carries the spike (9) is placed in a form-fitting manner.

2. Syringe in accordance with claim 1, **characterised in that** the outer casing surface of the ring element (13) has the shape of a double cone.

3. Syringe in accordance with claim 2, **characterised in that** the cone surfaces narrow towards the frontal faces of the ring element (13).

4. Syringe in accordance with one of the claims 1 to 3, **characterised in that** the surface of the membrane (7) that faces the spike (9) runs flush with the base of the recess (12).

5. Syringe in accordance with one of the claims 1 to 4, **characterised in that** in its area adjoining the plunger (3), the plunger rod (2) is equipped with a short external screw thread (10) which projects radially over the plunger shaft, and corresponding to this is an internal screw thread on that end of the syringe barrel (1) that faces away from the needle annex piece (4), or on a finger rest (11).

6. Syringe in accordance with one of the claims 1 to 5, **characterised in that** a filter disk (14) is arranged in the drilled through-hole (6), on that side of the spike (9) facing away from the membrane (7).

7. Syringe in accordance with one of the claims 1 to 6, **characterised in that** the needle annex piece (4), with a barrel-shaped connection piece (4.1.), encloses the needle side end - which is equipped with an external collar - of the syringe barrel (1).

8. Syringe in accordance with one of the claims 1 to 7, **characterised in that** arranged on the connection piece (4.1.) there is a ring projection (4.2) which projects radially inwards, and that on the external collar of the syringe barrel (1) two annular grooves (15, 16) are provided which are arranged one behind the other in an axial direction, wherein when the ring projection (4.2) engages into the first annular groove (15), the needle annex piece (4) holds the sealing plug (5) in a position such that an annular gap is formed between the interior of the external collar and the sealing plug (5), whilst when the ring projection (4.2) engages into the second annular groove (16), the sealing plug rests against the interior of the external collar to create a seal.

9. Syringe in accordance with one of the claims 1 to 8, **characterised in that** the connection piece (4.1) is equipped with several recesses which are arranged at regular intervals over the periphery, and which are designed as slits which run in an axial direction and extend to the free edge of the connection piece (4.1).

10. Syringe in accordance with one of the claims 1 to 9, **characterised in that** the needle annex piece (4) is fixed by means of a securing ring (17) which encompasses the connection piece (4.1).

11. Syringe in accordance with one of the claims 1 to 10, **characterised in that** the needle annex piece (4) has a cone (4.3), closed by a tip-cap (8), for fitting the needle.

12. Syringe in accordance with claim 11, **characterised in that** connected to the securing ring (17), via a connection web (18) which is designed as a predetermined breaking point, there is a securing cap (19) which, when the securing ring (17) is pushed onto the connection piece (4.1), encompasses the cone for the needle or the tip-cap (8) placed on it.

## Revendications

1. Seringue préremplie à usage médical, avec un corps de seringue (1), et un piston de seringue (3) monté dans celui-ci et déplaçable à l'aide d'une tige de piston (2), avec également, à l'extrémité côté canule du corps de seringue (1), un embout à canule (4) monté axialement et tenu par encliquetage (15, 16), qui entoure l'extrémité côté canule du corps de seringue (1), et un élément d'obturation (5) coaxial avec l'embout à canule (4), qui est inséré dans le corps de seringue (1), comporte un collet annulaire (5.1) en contact d'un côté avec la face frontale du corps de seringue (1) et de l'autre avec un épaulement annulaire de l'embout à canule (4), et est percé d'un trou débouchant (6) axial, une membrane (7) élastique disposée dans le trou débouchant (6) fermant celui-ci, au moins un perforateur (9) étant disposé en regard de la face de la membrane éloignée du corps de seringue (1), à faible distance de celle-ci, **caractérisée en ce que** l'élément d'obturation (5), sur sa face éloignée du corps de seringue (1) présente un évidement (12) en forme de trou borgne dans lequel est inséré par complémentarité de forme un élément annulaire (13) portant le perforateur (9).

2. Seringue selon la revendication 1, **caractérisée en ce que** la surface périphérique extérieure de l'élément annulaire (13) a la forme d'un cône double.

3. Seringue selon la revendication 2, **caractérisée en ce que** les surfaces de cône se rétrécissent en direction des faces frontales de l'élément annulaire.

4. Seringue selon une des revendications 1 à 3, **caractérisée en ce que** la surface de la membrane (7) tournée vers le perforateur (9) est en contact avec le fond de l'évidement (12).

5. Seringue selon une des revendications 1 à 4, **caractérisée en ce que** la tige de piston (2), dans sa partie voisine du piston (3), est pourvue d'un court filetage extérieur (10), en saillie radialement par rapport à la tige de piston, auquel est associé un filetage intérieur aménagé à l'extrémité du corps de seringue (1) éloignée de l'embout à canule (4) ou sur un élémént d'appui (11) pour les doigts.

6. Seringue selon une des revendications 1 à 5, **caractérisée en ce que** du côté du perforateur (9) éloigné de la membrane (7) un filtre-disque (14) est disposé dans le trou débouchant (6).

7. Seringue selon une des revendications 1 à 6, **caractérisée en ce que** l'embout à canule (4) entoure avec une partie de raccordement (4.1) en forme de manchon l'extrémité côté canule du corps de seringue (1) pourvue d'un collet extérieur.

8. Seringue selon une des revendications 1 à 7, **caractérisée en ce que** la partie de raccordement (4.1) comporte une saillie annulaire (4.2) qui s'avance radialement en direction de l'intérieur et **en ce qu'**il est prévu sur le collet extérieur du corps de seringue (1) deux gorges annulaires (15, 16), disposées l'une derrière l'autre dans la direction axiale, l'embout à canule (4), lorsque la saillie annulaire (4.2) est engagée dans la première gorge annulaire (15), tenant l'élément d'obturation (5) dans une position dans laquelle un espace annulaire est formé entre la face intérieure du collet extérieur et l'élément d'obturation (5), tandis que l'élément d'obturation, lorsque la saillie annulaire (4.2) est engagée dans la seconde gorge annulaire (16), est appliqué de manière étanche sur la face intérieure du collet extérieur.

9. Seringue selon une des revendications 1 à 8, **caractérisée en ce que** la partie de raccordement (4.1) est pourvue de plusieurs échancrures régulièrement réparties sur le pourtour, conformées en fentes axiales s'étendant jusqu'au bord libre de la partie de raccordement (4.1).

10. Seringue selon une des revendications 1 à 9, **caractérisée en ce que** l'embout à canule (4) est fixé par une bague de sécurité (17) qui entoure la partie de raccordement (4.1).

11. Seringue selon une des revendications 1 à 10, **caractérisée en ce que** l'embout à canule (4) présente un cône (4.3) pour recevoir la canule qui est fermé par un capuchon (8) d'embout.

12. Seringue selon la revendication 11, **caractérisée en ce qu'**à la bague de sécurité (17) est reliée par l'intermédiaire d'une bande de liaison (18) agencée en point de rupture à un capuchon de sécurité (19) qui entoure le cône pour la canule ou le capuchon (8) d'embout placé sur celui-ci lorsque la bague de sécurité (17) est enfilée sur la partie de raccordement (4.1).
